# EUROPEAN PATENT APPLICATION

(11) **EP 2 080 748 A2**
(43) Date of publication of application: **22.07.2009**
(21) Application number: 09150408.4
(22) Date of filing: 12.01.2009
(51) Int. Cl.: C07C 17/087, C07C 19/10

(54) **Hydrofluorination of 2-chloro-3,3,3-trifluoropropene to 2-chloro-1,1,1,2-tetrafluoropropane**

(30) Priority: 15.01.2008 US 21121 P; 18.12.2008 US 338466
(71) Applicant: Honeywell International Inc., Morristown, NJ 07962 (US)
(72) Inventor: Merkel, Daniel C., West Seneca, NY 14221 (US); Johnson, Robert C., Lancaster, NY 14806 (US); Tung, Hsuehsung, Getzville, NY 14068 (US)
(74) Representative: Buckley, Guy Julian

(57) **Abstract**

A process for making 2-ch1oro-1,1,1,2-tetrafluoropropane comprising hydrofluorinating 2-chloro-3,3,3-trifluoropropene in the presence of a catalyst selected from the group consisting of: SbC13, SbCl5, SbF₅, TiCl₄, SnCl₄, Cr₂O₃, and fluorinated Cr₂O₃.

## Description

### CROSS-REFERENCED APPLICATIONS

This application claims priority to U.S. Provisional Application No. 61/021121, filed on January 15, 2008, which is incorporated herein by reference in its entirety.

### BACKGROUND

### 1. Field

The present disclosure relates to a process for making 2-chloro-1,1,1,2-tetrafluoropropane. The present disclosure further relates to a process for making 2-chloro-1,1,1,2-tetrafluoropropane via hydrofluorination of 2-chloro-3,3,3-trifluoropropene with high single-pass conversion.

### 2. Description of the Related Art

The refrigerant and blowing agent 2,3,3,3-tetrafluoropropene (1234yf) is produced from the dehydrochlorination of 2-chloro-1,1,1,2-tetrafluoropropane (244bb). 244bb may be manufactured from 2-chloro-3,3,3-trifluoropropene (1233xf).

When conversion of 2-chloro-1,1,1,2-tetrafluoropropane from 2-chloro-3,3,3-trifluoropropene is low, 2-chloro-1,1,1,2-tetrafluoropropane and 2-chloro-3,3,3-trifluoropropene are present in admixture in product streams. 2-chloro-1,1,1,2-tetrafluoropropane and 2-chloro-3,3,3-trifluoropropene exhibit similar boiling points and azeotrope-like properties that make them difficult to separate via standard techniques such as conventional distillation.

One method of addressing the problem of low conversion is to increase recycle of product streams to the reactor so that additional conversion is obtained. The increased recycle would require process equipment to be increased in size and scale to maintain a desired level or product output, and, thus significantly increase manufacturing cost. In addition, the separation of components in the product stream is difficult.

It would be desirable to have a process for making 2-chloro-1,1,1,2-tetrafluoropropane from 2-chloro-3,3,3-trifluoropropene at higher single-pass conversion levels.

### SUMMARY

A process for making 2-chloro-1,1,1,2-tetrafluoropropane, comprising hydrofluorinating 2-chloro-3,3,3-trifluoropropene in the presence of a catalyst selected from the group consisting of SbCl₃, SbCl₅, SbF₅, TiCl₄, SnCl₄, Cr₂O₃, and fluorinated Cr₂O₃.

Preferably, the catalyst is activated using anhydrous hydrogen fluoride and possibly anhydrous chlorine. In addition, the catalyst is kept activated by the continuous or batch addition of chlorine (or similar oxidizing agent).

The hydrofluorination is vapor-phase fluorination. It is preferable that the catalyst for vapor-phase fluorination reaction is SbCl₅ supported on activated carbon. The vapor-phase fluorination reaction is carried out at a temperature of about 30° C to about 200° C, preferably about 50° C to about 120° C. The vapor-phase fluorination reaction is carried out at a pressure of about 5 psia to about 200 psia, preferably about 30 psia to about 175 psia.

The mole ratio of hydrogen fluoride to 2-chloro-3,3,3-trifluoropropene is from about 1:1 to about 30:1, preferably about 2:1 to about 15:1.

Alternatively, the hydrofluorination step is liquid-phase fluorination, where the reaction temperature is about 30-200°C, preferably about 50-120°C; and where the reaction pressure is about 15-200 psia, preferably about 50-175 psia; and where the mole ratio of hydrogen fluoride to 2-chloro-3,3,3-trifluoropropene is from about 1:1 to about 30:1, preferably about 2:1 to about 15:1.

### DETAILED DESCRIPTION OF THE PREFERRED EMBODIMENT

In the process of the present disclosure, selected catalysts are employed to enhance the single-pass conversion of 2-chloro-3,3,3-trifluoropropene to 2-chloro-1,1,1,2-tetrafluoropropane via HF addition across the double bond of 2-chloro-3,3,3-trifluoropropene. The catalysts are the following: SbCl₃, SbCl₅, SbF₅, TiCl₄, SnCl₄, Cr₂O₃, and fluorinated Cr₂O₃. Optionally, 2-chloro-3,3,3-trifluoropropene may additionally comprise HCl, which is carried over from previous step.

The hydrofluorination process may be carried out in a vapor phase or a liquid phase.

In vapor-phase hydrofluorination, HF (hydrogen fluoride gas) is fed continuously through the catalyst bed. After a short time with only the HF feed stream, 2-chloro-3,3,3-trifluoropropene is fed continuously through the catalyst bed at a ratio of about 1:1 to about 1:30 and preferably from about 1:2 to about 1:15 (2-chloro-3,3,3-trifluoropropene /HF mole ratio). The reaction between HF and 2-chloro-3,3,3-trifluoropropene is carried out at a temperature from about 30° C to about 200° C (preferably from about 50° C to about 120° C) and at a pressure of about 5 psia to about 200 psia (pounds per square inch absolute) (preferably from about 30 psia to about 175 psia). The catalyst may be supported on a substrate, such as on activated carbon, or may be unsupported or free-standing. In addition to activated carbon, useful catalyst supports include: alumina, fluorinated alumina, aluminum fluoride, alkaline earth metal oxides, fluorinated alkaline earth metals, zinc oxide, zinc fluoride, tin oxide, and tin fluoride. The catalyst may (or may not) have to be activated with anhydrous hydrogen fluoride HF (hydrogen fluoride gas) and/or Cl₂ (chlorine gas) before use depending on the state of the catalyst. If necessary, the catalyst can be kept activated by the continuous or batch addition of Cl₂ or a similar oxidizing agent.

In liquid phase hydrofluorination, the catalyst is charged in a liquid form to a reactor and optionally activated with HF. The activated catalyst is then heated to the desired reaction temperature of about 30° C to about 200° C (preferably from about 50° C to about 120° C) and the pressure is kept between about 15 psia to about 200 psia (preferably from about 50 psia to about 175 psia). After a short time with only HF feed, a 2-chloro-3,3,3-trifluoropropene feed stream is fed continuously through the catalyst at a ratio of about 1:1 to about 1:30 and preferably about 1:2 to about 1:15 (2-chloro-3,3,3-trifluoropropene /HF mole ratio). If necessary, the catalyst can be kept activated by the continuous or batch addition of Cl₂ or a similar oxidizing agent.

Enhanced or improved single-pass conversion of 2-chloro-3,3,3-trifluoropropene to 2-chloro-1,1,1,2-tetrafluoropropane is an important feature of the present disclosure. The hydrofluorination reaction is preferably carried out to attain a conversion of about 70% or more, preferably about 90% or more, and most preferably about 93% or more. Conversion is calculated by the number of moles of reactant (2-chloro-3,3,3-trifluoropropene) consumed divided by number of moles of reactant (2-chloro-3,3,3-trifluoropropene) fed to the reactor multiplied by 100. The selectivity for 2-chloro-1,1,1,2-tetrafluoropropane attained is preferably about 60% or more and most preferably about 80% or more. Selectivity is calculated by number of moles of product (2-chloro-1,1,1,2-tetrafluoropropane) formed divided by number of moles of reactant consumed.

Hydrofluorination is preferably carried out in a corrosion-resistant reaction vessel. Examples of corrosion-resistant materials are Hastelloy, Nickel, Incoloy, Inconel, Monel and fluoropolymer linings. The vessel may have a fixed or a fluidized catalyst bed, or contain liquid catalyst. If desired, inert gases such as nitrogen or argon may be employed in the reactor during operation.

The following are examples of the present disclosure and are not to be construed as limiting. Unless otherwise indicated, all percentages and parts are by weight.

### Examples

### Example 1

The vapor phase fluorination of the 2-chloro-3,3,3-trifluoropropene (1233xf) + HF → 2-chloro-1,1,1,2-tetrafluoropropane (244bb) was carried out. The fluorination catalyst for the experiment was 50 wt% SbCl₅ impregnated on 50 wt% Calgon PCB activated carbon.

Several kilograms of 50 weight% SbCl₅ on activated carbon were produced in the lab. The catalyst was first passed through a 10-mesh sieve to remove fines. A total of 2272.6 grams (or about 2800 cc) was charged to two 2-inch vapor-phase pipe reactors in series and installed in a sand bath for controlled heating.

The catalyst was activated by adding a minimum of a 5:1 mole ratio of HF to SbCl₅, followed by a Cl₂ addition of a minimum of a 3:1 1 mole ratio of Cl₂ to SbCl₅. Finally, a large excess of HF was passed through the catalyst bed for 2 hours.

The reaction was run using various cylinders of 2-chloro-3,3,3-trifluoropropene crude material as organic feed to produce 2-chloro-1,1,1,2-tetrafluoropropane. The reactor effluent was collected in the distillation column before removal of excess HF. During the experiment, a 93.5% conversion of 2-chloro-3,3,3-trifluoropropene was achieved. The maximum selectivity of 2-chloro-1,1,1,2-tetrafluoropropane achieved was 98.4% on a molar basis. The reaction ran continuously for 76.5 hrs without attempting catalyst regeneration with Cl₂. The catalyst began showing signs of deactivation after about 65 hours on-stream time. The experimental data and reaction conditions are shown below in Tables 1A and 1B.

| On-stream time (hrs) | T (°C) | P (Mpa) | Catalyst | 2-chloro-3,3,3-trifluoropropene feed rate (mmole/ min) | 2-chloro-3,3,3-trifluoropropene feed rate (g/hr) | HF feed rate (mmole/min) | HF feed rate (g/hr) | HF: 2-chloro-3,3,3-trifluoropropene mole ratio | Catalyst (ml) | Contact Time (sec) |
|---|---|---|---|---|---|---|---|---|---|---|
| 1-23 | 72 | 0.33 | SbCl₅/C | 12.0 | 95.3 | 185.2 | 222.3 | 15.5 | 2800 | 99 |
| 23-29 | 72 | 0.33 | SbCl₅/C | 18.3 | 145.2 | 215.5 | 258.6 | 11.8 | 2800 | 84 |
| 29-42 | 72 | 0.33 | SbCl₅/C | 23.4 | 186.0 | 241.9 | 290.3 | 10.3 | 2800 | 74 |
| 42-53 | 74 | 0.33 | SbCl₅/C | 30.2 | 240.4 | 275.9 | 331.1 | 9.1 | 2800 | 64 |
| 53-60 | 76 | 0.33 | SbCl₅/C | 39.3 | 322.1 | 317.5 | 381.0 | 8.1 | 2800 | 54 |
| 60-65.5 | 77 | 0.33 | SbCl₅/C | 48.1 | 394.6 | 400.7 | 480.8 | 8.3 | 2800 | 43 |
| 65.5-73.5 | 80 | 0.33 | SbCl₅/C | 51.1 | 408.2 | 404.5 | 485.4 | 7.9 | 2800 | 42 |
| 73.5-76.5 | 79 | 0.33 | SbCl₅/C | 33.9 | 281.2 | 355.3 | 426.4 | 10.5 | 2800 | 49 |

**Table 1B**

| On-stream | Reactor Effluent Composition (GC area%) | | |
|---|---|---|---|
| Time (hrs) | 2-chloro-1,1,1,2-tetrafluoropropane | 2-chloro-3,3,3-trifluoropropene (1233xf) | 2,3-Dichloro-3,3-difluoropropene (1232xf) |
| 1-23 | 0.31 | 82.41 | 17.03 |
| 23-29 | 0.31 | 82.41 | 17.03 |
| 29-42 | 0.31 | 82.41 | 17.03 |
| 42-53 | 0.31 | 82.41 | 17.03 |
| 53-60 | 31.08 | 68.92 | 0 |
| 60-65.5 | 31.08 | 68.92 | 0 |
| 65.5-73.5 | 21.41 | 77.36 | 0 |
| 73.5-76.5 | 38.86 | 61.09 | 0 |

**Table 1C**

| On-stream | Reactor Effluent Composition (GC area%) | | | | |
|---|---|---|---|---|---|
| Time (hrs) | 2-chloro-1,1,1,2-tetrafluoropropane | 2-chloro-3,3,3-trifluoropropene | 2,3-Dichloro-3,3-difluoropropene | 1,2-dichloro-3,3,3-trifluoropropene | others |
| 1-23 | 86.2 | 7.5 | 0.2 | 0.1 | 6.0 |
| 23-29 | 91.7 | 5.1 | 0.2 | 0.1 | 3.0 |
| 29-42 | 92.1 | 4.8 | 0.2 | 0.0 | 2.8 |
| 42-53 | 91.3 | 5.9 | 0.1 | 0.0 | 2.7 |
| 53-60 | 92.0 | 7.1 | 0.0 | 0.0 | 1.0 |
| 60-65.5 | 90.0 | 8.9 | 0.0 | 0.0 | 1.2 |
| 65.5-73.5 | 87.1 | 10.2 | 0.0 | trace | 2.7 |
| 73.5-76.5 | 86.3 | 11.6 | 0.0 | 0.0 | 2.2 |

**Table 1D**

| On-stream | Feed composition (GC area%) | | | | | |
|---|---|---|---|---|---|---|
| 1233xf | 1232xf | Selectivities (molar basis assuming GC area% = wt%) | | | | |
| Conversion | Conversion | 1234yf/245cb | 244bb | 1232xf | 1223xd | others |
| 89.9 | 99.0 | 3.9 | 92.6 | NA | 0.1 | 3.2 |
| 93.2 | 98.8 | 1.6 | 96.4 | NA | 0.0 | 1.8 |
| 93.5 | 99.0 | 1.2 | 96.6 | NA | 0.0 | 2.0 |
| 92.0 | 99.3 | 1.1 | 96.9 | NA | 0.0 | 1.9 |
| 83.8 | NA | 1.0 | 98.4 | 0.0 | 0.0 | 0.6 |
| 82.8 | NA | 1.2 | 98.1 | 0.0 | 0.0 | 0.7 |
| 81.5 | NA | 1.5 | 97.8 | 0.0 | 0.0 | 0.7 |
| 75.1 | NA | 3.1 | 95.5 | 0.1 | 0.0 | 1.6 |

### Example 2

The liquid phase fluorination of the 2-chloro-3,3,3-trifluoropropene (1233xf) + HF → 2-chloro-1,1,1,2-tetrafluoropropane (244bb). The fluorination catalyst for the experiment was SbCl₅.

About 6100 grams of SbCl₅ were contained in a Teflon™-lined liquid phase reactor (Teflon is a trademark of E.I. duPont de Nemours & Co) equipped with a 2-inch ID (inside diameter) packed column and a condenser. The reactor is 2.75-inch ID x 36-inch L (length). A large excess of Cl₂ was first added to the reactor to ensure that the catalyst was in a pentavalent state. The reactor was heated to about 85° C - 87° C. HF feed was started first. When 1.3 lbs (pounds) of HF had been added the 2-chloro-3,3,3-trifluoropropene feed was started. The purity of the 2-chloro-3,3,3-trifluoropropene feed stock was about 98 GC area % (gas chromatograph). The experiment ran continuously for 71 hours. For this run, chlorine was fed batchwise about every 4 hours throughout the run to keep the catalyst active. The HF and 2-chloro-3,3,3-trifluoropropene feeds were varied during the run. The feeds averaged 0.495 lbs/hr HF, and 0.408 lbs/hr 2-chloro-3,3,3-trifluoropropene (chlorine was 5.4% by weight of organic) for a 7.9/1 ratio of HF/2-chloro-3,3,3-trifluoropropene, and 135 seconds residence time at the beginning of the run. In the middle of the run, the feeds averaged 0.843 lbs/hr HF (pounds/hour) and 0.66 lbs/hr 2-chloro-3,3,3-trifluoropropene (chlorine was 3.3% by weight of organic) for a 8.33/1 ratio of HF/2-chloro-3,3,3-trifluoropropene, and 80 seconds residence time. For the end of the run, the rate was increased. The feeds for this period averaged 1.42 lbs/hr HF and 1.24 lbs/hr 2-chloro-3,3,3-trifluoropropene (chlorine was 2 % by weight of organic) for a 7.5/1 ratio of HF/2-chloro-3,3,3-trifluoropropene, and 47 seconds residence time. The level of unreacted 2-chloro-3,3,3-trifluoropropene appeared to increase late in the run, which could have been the result of lower Cl₂ level or shorter residence time.

The reactor temperature range for the experiment was 78 - 91°C and the pressure range was 85 psig - 115 psig (pounds per square inch gauge). The organic crude material collected from the run was run on a gas chromatograph and had the following GC analysis.

The organic phase exhibited the following when analyzed by using a gas chromatograph:

| **GC Area%** | |
|---|---|
| 1,1,1,2,2-pentafluoropropane | = 11.80 |
| 2-chloro-1,1,1,2-tetrafluoropropane | = 82.87 |
| 2-chloro-3,3,3-trifluoropropene | = 1.14 |
| 2-Chloro-1,1,1,3,3-pentafluoropropane | = 0.52 |
| 2,3-dichcloro-3,3-difluoropropene | = 0.20 |
| 1,2-dichloro-3,3,3-trifluoropropene | = 2.44 |

The following Table 2 contains the 2-chloro-3,3,3-trifluoropropene conversion and product selectivity data:

**Table 2**

| (Conversion and Selectivity on a Molar Basis) | | | | | | | |
|---|---|---|---|---|---|---|---|
| hours | 1,1,1,2,2-pentafluoro propane | 2-chloro-1,1,1,2-tetrafluoropropane | 2-chloro-3,3,3-trifluoropropene | 2-Chloro-1,1,1,3,3-pentafluoropropane | 1,2-dichloro-3,3,3-trifluoropropene | Others | °C |
| elapsed Time | Selectivity | Selectivity | Conversion | Selectivity | Selectivity | Selectivity | Temp |
| 2 | 64.8 | 24.2 | 99.3 | 0.0 | 0.0 | 11.1 | 87.1 |
| 3 | 68.2 | 24.2 | 99.2 | 0.9 | 4.8 | 1.9 | 90.5 |
| 4 | 67.5 | 24.3 | 99.8 | 0.6 | 3.6 | 3.9 | 90.2 |
| 5 | 64.6 | 30.0 | 99.9 | 1.2 | 3.1 | 1.1 | 90.4 |
| 6 | 67.4 | 27.2 | 99.8 | 1.2 | 3.1 | 1.0 | 85.7 |
| 8 | 82.8 | 15.6 | 99.7 | 0.4 | 0.8 | 0.5 | 78.9 |
| 9 | 78.5 | 20.2 | 99.9 | 0.3 | 0.6 | 0.4 | 78.9 |
| 10 | 65.4 | 32.3 | 99.6 | 0.6 | 1.0 | 0.6 | 83.2 |
| 11 | 61.8 | 35.8 | 99.0 | 0.6 | 1.0 | 0.7 | 78.5 |
| 12 | 64.8 | 33.7 | 99.3 | 0.5 | 0.6 | 0.4 | 79.6 |
| 13.5 | 61.6 | 37.0 | 99.8 | 0.5 | 0.5 | 0.4 | 80.9 |
| 14 | 62.1 | 36.5 | 99.7 | 0.5 | 0.5 | 0.5 | 81.3 |
| 15 | 61.9 | 36.8 | 99.6 | 0.5 | 0.4 | 0.4 | 78.9 |
| 16 | 29.1 | 68.3 | 99.5 | 1.3 | 0.6 | 0.7 | 86.9 |
| 17 | 30.5 | 67.3 | 98.6 | 1.2 | 0.5 | 0.5 | 88.5 |
| 18 | 24.4 | 73.0 | 98.8 | 1.5 | 0.6 | 0.5 | 84.5 |
| 19 | 31.0 | 66.1 | 98.3 | 1.6 | 0.7 | 0.5 | 87.5 |
| 20 | 28.7 | 66.8 | 99.8 | 2.5 | 1.2 | 0.9 | 84.5 |
| 21 | 33.8 | 62.9 | 99.7 | 1.8 | 0.9 | 0.6 | 86.9 |
| 22 | 51.6 | 46.6 | 99.5 | 0.9 | 0.5 | 0.5 | 86.6 |
| 23 | 54.3 | 45.1 | 99.7 | 0.2 | 0.1 | 0.2 | 85.6 |
| 24 | 28.3 | 70.1 | 99.5 | 0.8 | 0.4 | 0.4 | 86.9 |
| 25 | 23.0 | 74.8 | 99.0 | 1.1 | 0.6 | 0.5 | 86.4 |
| 26 | 16.0 | 76.2 | 98.3 | 3.6 | 2.8 | 1.3 | 86.3 |
| 27 | 20.8 | 73.2 | 98.3 | 2.7 | 2.1 | 1.2 | 85.5 |
| 28 | 12.0 | 78.3 | 99.0 | 3.2 | 2.7 | 3.8 | 87 |
| 29 | 11.9 | 79.8 | 98.7 | 2.1 | 2.0 | 4.2 | 87.9 |
| 30 | 11.0 | 80.8 | 98.6 | 2.1 | 2.0 | 4.2 | 87.1 |
| 31 | 13.9 | 81.7 | 98.2 | 0.8 | 1.0 | 2.6 | 86.2 |
| 32 | 10.2 | 86.6 | 99.3 | 0.4 | 0.7 | 2.2 | 85.9 |
| 33 | 9.4 | 87.9 | 98.8 | 0.2 | 1.4 | 1.0 | 85.5 |
| 34 | 12.6 | 85.8 | 98.5 | 0.1 | 0.7 | 0.8 | 85.4 |
| 35 | 15.1 | 83.6 | 98.1 | 0.1 | 0.5 | 0.7 | 85.3 |
| 36 | 4.3 | 92.3 | 98.2 | 0.1 | 2.2 | 1.1 | 85.2 |
| 37 | 4.7 | 92.3 | 97.9 | 0.1 | 1.8 | 1.2 | 84.9 |
| 38 | 4.8 | 92.7 | 97.9 | 0.1 | 1.5 | 1.0 | 85.4 |
| 39.5 | 8.6 | 89.5 | 97.8 | 0.0 | 0.1 | 1.8 | 85.1 |
| 41.7 | 17.1 | 81.4 | 98.1 | 0.0 | 0.6 | 0.9 | 85 |
| 42.7 | 14.0 | 85.7 | 97.8 | 0.0 | 0.1 | 0.3 | 83.6 |
| 44.7 | 20.4 | 79.1 | 98.1 | 0.0 | 0.0 | 0.4 | 80.6 |
| 46 | 6.0 | 92.5 | 98.3 | 0.0 | 0.9 | 0.5 | 84.2 |
| 47.5 | 6.1 | 91.1 | 99.7 | 0.0 | 1.5 | 1.3 | 86.2 |
| 48 | 6.2 | 91.5 | 99.9 | 0.0 | 1.3 | 1.0 | 87.1 |
| 49 | 10.6 | 86.8 | 98.9 | 0.0 | 1.7 | 0.9 | 86.9 |
| 50 | 7.2 | 91.0 | 98.1 | 0.0 | 1.1 | 0.7 | 86.6 |
| 51 | 10.9 | 88.4 | 97.7 | 0.0 | 0.3 | 0.4 | 86.7 |
| 52 | 13.9 | 82.9 | 98.7 | 0.0 | 2.3 | 0.9 | 89.3 |
| 53 | 12.7 | 86.0 | 97.9 | 0.0 | 0.6 | 0.8 | 87.5 |
| 54 | 9.5 | 89.4 | 97.7 | 0.0 | 0.5 | 0.6 | 88 |
| 55 | 6.6 | 92.2 | 98.3 | 0.0 | 0.6 | 0.7 | 87.1 |
| 56 | 6.8 | 89.6 | 98.1 | 0.0 | 2.7 | 1.0 | 87.4 |
| 57 | 7.5 | 91.1 | 97.6 | 0.0 | 0.7 | 0.7 | 87.7 |
| 58.1 | 5.4 | 91.6 | 99.8 | 0.1 | 1.4 | 1.6 | 87.6 |
| 60 | 6.2 | 92.7 | 98.8 | 0.0 | 0.2 | 0.9 | 87.8 |
| 61 | 5.6 | 93.5 | 100.0 | 0.0 | 0.1 | 0.7 | 87.8 |
| 62 | 7.6 | 89.3 | 99.6 | 0.0 | 2.0 | 1.0 | 87.7 |
| 63 | 7.8 | 89.1 | 97.9 | 0.1 | 2.3 | 0.7 | 87.9 |
| 64 | 9.0 | 90.2 | 99.3 | 0.0 | 0.3 | 0.5 | 87.7 |
| 65.3 | 0.0 | 99.4 | 96.9 | 0.0 | 0.2 | 0.3 | 88 |
| 66 | 5.2 | 91.7 | 99.7 | 0.1 | 2.0 | 1.0 | 87.2 |
| 69 | 3.3 | 96.2 | 96.1 | 0.1 | 0.2 | 0.3 | 88 |
| 70 | 3.0 | 95.1 | 95.3 | 0.1 | 1.3 | 0.5 | 87.9 |
| 71 | 2.8 | 95.4 | 96.8 | 0.0 | 0.4 | 1.4 | 88.5 |

### Example 3

Example 3 used the same equipment as Example 2.

About 5615 grams of SbCl₅ were contained in the same reactor as that of Example 2. The reactor was heated to about 85° C - 87° C. HF feed was started first. After about 1.5 lbs of HF had been added, the 2-chloro-3,3,3-trifluoropropene feed was started. The purity of the 2-chloro-3,3,3-trifluoropropene feed stock was about 97.3 GC area%. The experiment ran continuously for 71 hours. For this run, Cl₂ was fed batchwise about every 4 hours throughout the run to keep the catalyst active.

The Run number (Run#) for this experiment was 36b. Conversion was immediately above 98%, and remained that way throughout the rest of the run (through Friday shutdown). The catalyst charge was left hot over the weekend, and operation resumed on Monday (now called Run #37), and similar high conversion was observed throughout the week. About 123 pounds of acid-free 2-chloro-1,1,1,2-tetrafluoropropane crude was collected between runs #36b and its continuation as Run #37 the following week. The times of chlorine addition are noted on the data for Run #37 in the appendix - it can be seen that there is a significant increase in the 1,2-dichloro-3,3,3-trichloropropene on the samples immediately (typically about one hour) after this addition, which then decreases.

The reactor temperature range for the experiment was 78° C - 86° C and the pressure range was 70 psig - 105 psig. The organic crude material collected from the run was run on a gas chromatograph and exhibited the following GC analysis.

The 2-chloro-1,1,1,2-tetrafluoropropane crude product collected from Run# 36b and #37 had the following analysis by GC:

| **Area%** | |
|---|---|
| 1,1,1,2,2-pentafluoropropane | = 4.48 |
| 2-chloro-1,1,1,2-tetrafluoropropane | = 91.59 |
| 2-chloro-3,3,3-trifluoropropene | = 2.10 |
| 2-Chloro-1,1,1,3,3-pentafluoropropane | = 0.21 |
| 2,3-Dichloro-3,3-difluoropropene | = 0.17 |
| 1,2-dichloro-3,3,3-trifluoropropene | = 1.13 |

The following Tables 3A and 3B set forth the 2-chloro-3,3,3-trifluoropropene conversion and product selectivity data.

**Table 3A**

| **(Run #36b, Conversion and Selectivity on a Molar Basis)** | | | | | | | | |
|---|---|---|---|---|---|---|---|---|
| Hours elapsed Time | GasBag # | Temp (°C) | Selectivity 1,1,1,2,2-pentafluoropropane | Selectivity 2-chloro-1,1,1,2-tetrafluoropropane | Conversion % 2-chloro-3,3,3-trifluoropropene | Selectivity 2-Chloro-1,1,1,3,3-pentafluoropropane | Selectivity 2,3-Dichloro-3,3-difluoropropene | Selectivity 1,2-dichloro-3,3,3-trifluoropropene |
| 35.3 | 36-b3 | 84.1 | 0.64 | 0.26 | 98.69 | 0.01 | 0.02 | 0.06 |
| 36.3 | 36-b4 | 85 | 0.43 | 0.54 | 98.70 | 0.01 | 0.00 | 0.01 |
| 37.3 | 36-b5 | 85.2 | 0.55 | 0.41 | 98.94 | 0.01 | 0.01 | 0.02 |
| 38.6 | 36-b6 | 85.6 | 0.44 | 0.51 | 98.71 | 0.01 | 0.00 | 0.02 |
| 39.2 | 36-b7 | 83.6 | 0.30 | 0.63 | 97.77 | 0.01 | 0.01 | 0.03 |
| 39.7 | 36-b8 | 85.5 | 0.25 | 0.70 | 97.26 | 0.01 | 0.01 | 0.02 |
| 40.8 | 36-b9 | 86.9 | 0.36 | 0.59 | 98.08 | 0.01 | 0.00 | 0.03 |
| 41.6 | 36-b10 | 83 | 0.55 | 0.44 | 98.94 | 0.00 | 0.00 | 0.01 |
| 42.4 | 36-b11 | 85.9 | 0.40 | 0.58 | 98.40 | 0.00 | 0.00 | 0.01 |
| 43.4 | 36-b12 | 85.3 | 0.37 | 0.61 | 98.42 | 0.00 | 0.00 | 0.00 |
| 44.75 | 36-b13 | 83.1 | 0.29 | 0.70 | 98.37 | 0.00 | 0.01 | 0.01 |
| 45.5 | 36-b14 | 80 | 0.23 | 0.76 | 98.44 | 0.00 | 0.00 | 0.00 |
| 46.5 | 36-b15 | 81.7 | 0.21 | 0.76 | 98.40 | 0.00 | 0.00 | 0.01 |
| 47.5 | 36-b16 | 81.3 | 0.19 | 0.79 | 98.21 | 0.00 | 0.00 | 0.01 |

**Table 3B**

| **(Run #37, Conversion and Selectivity on a Molar Basis)** | | | | | | | | | |
|---|---|---|---|---|---|---|---|---|---|
| Hours elapsed Time | Gas Bag # | Temp (°C) | HF/Org mole ratio | molar selectivity 1,1,1,2,2-pentafluor opropane | molar selectivity 2-chloro-1,1,1,2-tetrafluoropropane | molar Conversion 2-chloro-3,3,3-trifluoropropene | molar selectivity 2-Chloro-1,1,1,3,3-pentafluoroprorpane | molar selectivity 2,3-Dichloro-3,3-difluoropropene | molar selectivity -1,2-dichloro-3,3,3-trifluoropropene |
| 1.3 | 1 | 87.5 | 11.84 | 0.16 | 0.82 | 98 | 0.002 | 0.002 | 0.005 |
| 2.4 | 2 | 85.2 | 6.09 | 0.10 | 0.89 | 98 | 0.002 | 0.002 | 0.005 |
| 3.25 | 3 | 86.5 | 5.49 | 0.13 | 0.84 | 98 | 0.003 | 0.002 | 0.013 |
| 4.4 | 4 | 83.2 | 7.03 | 0.10 | 0.88 | 98 | 0.003 | 0.002 | 0.017 |
| 5.4 | 5 | 83.3 | 8.80 | 0.10 | 0.88 | 98 | 0.002 | 0.001 | 0.008 |
| 6.4 | 6 | 81.5 | 8.00 | 0.08 | 0.90 | 98 | 0.002 | 0.001 | 0.004 |
| 7.4 | 7 | 79.9 | 20.74 | 0.08 | 0.90 | 98 | 0.002 | 0.001 | 0.008 |
| 8.3 | 8 | 80 | 7.54 | 0.07 | 0.92 | 98 | 0.001 | 0.001 | 0.004 |
| 9.3 | 9 | 81.3 | 4.44 | 0.09 | 0.90 | 98 | 0.001 | 0.001 | 0.003 |
| 10.3 | 10 | 85.1 | 3.57 | 0.11 | 0.88 | 98 | 0.001 | 0.001 | 0.003 |
| 11.3 | 11 | 88 | 4.64 | 0.15 | 0.83 | 98 | 0.002 | 0.002 | 0.015 |
| 12.6 | 12 | 85.5 | 5.03 | 0.14 | 0.85 | 98 | 0.001 | 0.002 | 0.004 |
| 13.4 | 13 | 85.3 | 4.68 | 0.10 | 0.89 | 98 | 0.001 | 0.002 | 0.003 |
| 14.3 | 14 | 82.8 | 5.08 | 0.08 | 0.91 | 98 | 0.001 | 0.002 | 0.003 |
| 15.3 | 15 | 83.7 | 5.63 | 0.09 | 0.89 | 98 | 0.002 | 0.001 | 0.012 |
| 16.25 | 16 | 84.2 | 7.21 | 0.08 | 0.91 | 98 | 0.001 | 0.001 | 0.004 |
| 17.4 | 17 | 86.1 | 7.86 | 0.09 | 0.91 | 98 | 0.001 | 0.001 | 0.003 |
| 18.3 | 18 | 85.7 | 8.33 | 0.07 | 0.92 | 98 | 0.001 | 0.001 | 0.002 |
| 19.3 | 19 | 86 | 7.38 | 0.09 | 0.88 | 98 | 0.003 | 0.002 | 0.018 |
| 20.3 | 20 | 87.8 | 8.27 | 0.09 | 0.90 | 98 | 0.002 | 0.001 | 0.003 |
| 21.4 | 21 | 83.4 | 10.48 | 0.08 | 0.88 | 98 | 0.002 | 0.003 | 0.003 |
| 22.4 | 22 | 88.7 | 18.21 | 0.08 | 0.91 | 98 | 0.001 | 0.001 | 0.003 |
| 23.3 | 23 | 83 | 9.26 | 0.08 | 0.90 | 98 | 0.002 | 0.001 | 0.007 |
| 24.3 | 24 | 82.9 | 7.46 | 0.06 | 0.93 | 98 | 0.001 | 0.001 | 0.004 |
| 25.3 | 25 | 81.3 | 7.19 | 0.06 | 0.94 | 98 | 0.001 | 0.001 | 0.003 |
| 26.3 | 26 | 83.9 | 8.05 | 0.05 | 0.94 | 98 | 0.001 | 0.001 | 0.003 |
| 27.3 | 27 | 81.9 | 7.61 | 0.06 | 0.92 | 98 | 0.003 | 0.001 | 0.016 |
| 28.3 | 28 | 83.8 | 6.90 | 0.06 | 0.93 | 98 | 0.001 | 0.001 | 0.003 |
| 29.3 | 29 | 83.9 | 7.18 | 0.07 | 0.93 | 98 | 0.001 | 0.001 | 0.003 |
| 30.3 | 30 | 85 | 6.23 | 0.08 | 0.92 | 97 | 0.001 | 0.001 | 0.003 |
| 31.3 | 31 | 83.4 | 6.27 | 0.06 | 0.91 | 98 | 0.003 | 0.002 | 0.016 |
| 32.3 | 32 | 82.8 | 6.66 | 0.05 | 0.94 | 98 | 0.001 | 0.001 | 0.004 |
| 34.3 | 33 | 85.2 | 5.64 | 0.06 | 0.93 | 98 | 0.001 | 0.001 | 0.003 |
| 35.3 | 34 | 86 | 5.30 | 0.07 | 0.91 | 97 | 0.001 | 0.001 | 0.008 |
| 36.3 | 35 | 84.9 | 7.23 | 0.07 | 0.92 | 97 | 0.001 | 0.001 | 0.003 |
| 37.5 | 36 | 80.7 | 7.58 | 0.06 | 0.94 | 98 | 0.001 | 0.001 | 0.002 |
| 38.3 | 37 | 82.2 | 5.81 | 0.03 | 0.97 | 98 | 0.001 | 0.002 | 0.003 |
| 39.25 | 38 | 81.9 | 6.32 | 0.04 | 0.94 | 98 | 0.002 | 0.002 | 0.013 |
| 40.25 | 39 | 82 | 6.32 | 0.04 | 0.95 | 98 | 0.002 | 0.001 | 0.006 |
| 41.5 | 40 | 81.4 | 5.77 | 0.04 | 0.94 | 98 | 0.001 | 0.001 | 0.004 |
| 42.5 | 41 | 81 | 6.20 | 0.04 | 0.95 | 98 | 0.001 | 0.001 | 0.003 |
| 43.8 | 42 | 81.4 | 8.14 | 0.03 | 0.96 | 98 | 0.001 | 0.001 | 0.003 |
| 44.7 | 43 | 80.7 | 8.14 | 0.03 | 0.97 | 98 | 0.000 | 0.001 | 0.001 |
| 45.5 | 44 | 80.9 | 6.88 | 0.03 | 0.97 | 98 | 0.000 | 0.000 | 0.001 |
| 47 | 45 | 82.8 | 7.16 | 0.14 | 0.84 | 98 | 0.003 | 0.002 | 0.010 |
| 47.8 | 46 | 82.3 | 7.70 | 0.03 | 0.96 | 98 | 0.001 | 0.000 | 0.002 |
| 48.8 | 47 | 82.3 | 7.18 | 0.03 | 0.97 | 98 | 0.000 | 0.000 | 0.001 |
| 49.8 | 48 | 82.5 | 6.67 | 0.03 | 0.97 | 98 | 0.000 | 0.000 | 0.001 |
| 50.8 | 49 | 82.8 | 6.68 | 0.03 | 0.95 | 98 | 0.002 | 0.001 | 0.013 |
| 51.8 | 50 | 82.7 | 6.84 | 0.03 | 0.97 | 98 | 0.001 | 0.000 | 0.002 |
| 53 | 51 | 81.3 | 8.09 | 0.03 | 0.97 | 98 | 0.000 | 0.000 | 0.001 |
| 54.3 | 52 | 79.8 | 8.60 | 0.03 | 0.97 | 98 | 0.000 | 0.000 | 0.001 |
| 54.8 | 53 | 81.2 | 4.22 | 0.03 | 0.95 | 98 | 0.002 | 0.001 | 0.015 |
| 56 | 54 | 81.6 | 6.75 | 0.03 | 0.97 | 98 | 0.000 | 0.000 | 0.002 |
| 56.8 | 55 | 83.6 | 6.45 | 0.03 | 0.97 | 97 | 0.000 | 0.000 | 0.001 |
| 57.8 | 56 | 84.9 | 7.03 | 0.03 | 0.97 | 97 | 0.000 | 0.000 | 0.001 |
| 58.8 | 57 | 81.5 | 7.11 | 0.04 | 0.95 | 98 | 0.001 | 0.001 | 0.009 |
| 59.8 | 58 | 82.8 | 7.11 | 0.03 | 0.97 | 98 | 0.000 | 0.000 | 0.002 |
| 60.8 | 59 | 81.1 | 6.99 | 0.02 | 0.98 | 98 | 0.000 | 0.000 | 0.001 |
| 63 | 60 | 84.2 | 7.51 | 0.02 | 0.96 | 98 | 0.001 | 0.001 | 0.010 |
| 64 | 61 | 84 | 8.79 | 0.02 | 0.97 | 98 | 0.001 | 0.000 | 0.004 |
| 65 | 62 | 82.9 | 8.79 | 0.02 | 0.97 | 98 | 0.000 | 0.000 | 0.001 |
| 66 | 63 | 82.6 | 6.44 | 0.02 | 0.98 | 98 | 0.000 | 0.000 | 0.001 |
| 67 | 64 | 83.2 | 7.33 | 0.03 | 0.94 | 98 | 0.005 | 0.001 | 0.015 |
| 68.25 | 65 | 82.1 | 5.28 | 0.04 | 0.95 | 98 | 0.002 | 0.001 | 0.004 |
| 69 | 66 | 83 | 7.22 | 0.03 | 0.96 | 98 | 0.001 | 0.000 | 0.002 |
| 70 | 67 | 82.6 | 6.63 | 0.03 | 0.97 | 98 | 0.000 | 0.000 | 0.001 |
| 71 | 68 | 82.5 | 4.98 | 0.03 | 0.96 | 98 | 0.001 | 0.000 | 0.001 |
| 72 | 69 | 82.1 | 5.28 | 0.03 | 0.95 | 98 | 0.002 | 0.001 | 0.020 |
| 73 | 70 | 81.1 | 4.75 | 0.02 | 0.97 | 98 | 0.000 | 0.001 | 0.002 |
| 74.25 | 71 | 82.2 | 4.77 | 0.03 | 0.97 | 98 | 0.000 | 0.000 | 0.001 |
| 75.1 | 72 | 87.1 | 5.20 | 0.03 | 0.97 | 98 | 0.000 | 0.000 | 0.001 |
| 75.8 | 73 | 81.3 | 4.09 | 0.03 | 0.95 | 98 | 0.001 | 0.001 | 0.016 |
| 78 | 74 | 81.4 | 8.64 | 0.02 | 0.97 | 98 | 0.000 | 0.000 | 0.002 |
| 79.1 | 75 | 80.4 | 7.16 | 0.02 | 0.98 | 98 | 0.000 | 0.000 | 0.001 |
| 80 | 76 | 83.2 | 6.11 | 0.03 | 0.96 | 98 | 0.002 | 0.000 | 0.008 |
| 81.1 | 77 | 83.4 | 6.21 | 0.02 | 0.97 | 98 | 0.000 | 0.000 | 0.002 |
| 83.25 | 78 | 84 | 7.41 | 0.02 | 0.97 | 97 | 0.000 | 0.000 | 0.001 |
| 84.3 | 79 | 85.5 | 7.17 | 0.02 | 0.96 | 98 | 0.002 | 0.000 | 0.018 |
| 85 | 80 | 84.4 | 12.16 | 0.02 | 0.98 | 98 | 0.001 | 0.000 | 0.003 |
| 86 | 81 | 82.1 | 9.15 | 0.02 | 0.98 | 98 | 0.000 | 0.000 | 0.001 |
| 87 | 82 | 81.9 | 7.69 | 0.02 | 0.98 | 98 | 0.001 | 0.000 | 0.001 |
| 88.4 | 83 | 82.4 | 4.58 | 0.02 | 0.94 | 98 | 0.007 | 0.001 | 0.031 |
| 89 | 84 | 83.4 | 9.46 | 0.02 | 0.97 | 98 | 0.001 | 0.000 | 0.004 |
| 90 | 85 | 81.5 | 7.22 | 0.02 | 0.98 | 98 | 0.001 | 0.000 | 0.001 |
| 91.2 | 86 | 82.5 | 7.09 | 0.02 | 0.98 | 98 | 0.000 | 0.000 | 0.001 |
| 92 | 87 | 83.4 | 7.49 | 0.01 | 0.97 | 98 | 0.001 | 0.001 | 0.015 |
| 93 | 88 | 82.4 | 6.60 | 0.02 | 0.98 | 98 | 0.001 | 0.000 | 0.002 |
| 94 | 89 | 82.3 | 6.25 | 0.01 | 0.97 | 98 | 0.002 | 0.000 | 0.004 |
| 95 | 89. 5 | 82.4 | 6.53 | 0.02 | 0.98 | 98 | 0.000 | 0.000 | 0.001 |
| 96.5 | 90 | 83.1 | 4.76 | 0.02 | 0.97 | 96 | 0.002 | 0.001 | 0.016 |
| 97 | 91 | 82.6 | 5.01 | 0.01 | 0.97 | 95 | 0.003 | 0.001 | 0.021 |
| 97.75 | 92 | 81 | 7.29 | 0.01 | 0.98 | 97 | 0.001 | 0.001 | 0.015 |
| 98.8 | 93 | 83.1 | 6.74 | 0.02 | 0.98 | 98 | 0.000 | 0.001 | 0.012 |
| 100.2 | 94 | 82.6 | 9.05 | 0.01 | 0.98 | 98 | 0.002 | 0.000 | 0.004 |
| 101.1 | 95 | 83.3 | 5.98 | 0.02 | 0.97 | 98 | 0.000 | 0.000 | 0.003 |
| 102.3 | 96 | 85.5 | 5.11 | 0.02 | 0.97 | 97 | 0.000 | 0.000 | 0.001 |
| 103.1 | 97 | 82.7 | 5.22 | 0.02 | 0.97 | 97 | 0.001 | 0.001 | 0.007 |
| 104 | 98 | 82.4 | 5.11 | 0.02 | 0.98 | 97 | 0.000 | 0.000 | 0.001 |
| 107 | 99 | 80.4 | 5.87 | 0.02 | 0.98 | 98 | 0.000 | 0.000 | 0.001 |
| 109 | 100 | 82.6 | 7.98 | 0.02 | 0.97 | 98 | 0.000 | 0.000 | 0.001 |
| 110 | 101 | 93.3 | 5.30 | 0.03 | 0.85 | 97 | 0.000 | 0.001 | 0.001 |
| 111 | 102 | 88.8 | 4.86 | 0.03 | 0.82 | 85 | 0.000 | 0.001 | 0.001 |
| 112 | 103 | 89.4 | 5.74 | 0.03 | 0.96 | 82 | 0.000 | 0.000 | 0.000 |
| 113 | 104 | 82.8 | 10.71 | 0.02 | 0.97 | 96 | 0.000 | 0.000 | 0.000 |
| 114 | 105 | 82.1 | 9.83 | 0.01 | 0.97 | 97 | 0.000 | 0.001 | 0.001 |

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances that fall within the scope of the appended claims.

## Claims

1. A process for making 2-chloro-1,1,1,2-tetrafluoropropane, comprising hydrofluorinating 2-chloro-3,3,3-trifluoropropene in the presence of a catalyst selected from the group consisting of SbCl₃, SbCl₅, SbF₅, TiCl₄, SnCl₄, Cr₂O₃, and fluorinated Cr₂O₃.

2. The process of claim 1, wherein the catalyst is in bulk form or support.

3. The process of claim 2, wherein the support is at least one support selected from the group consisting of: carbon, alumina, fluorinated alumina, aluminum fluoride, alkaline earth metal oxides, fluorinated alkaline earth metals, zinc oxide, zinc fluoride, tin oxide, and tin fluoride.

4. The process of claim 1, wherein the catalyst is activated using anhydrous hydrogen fluoride, anhydrous chlorine or chlorine.

5. The process of claim 1, wherein 2-chloro-3,3,3-trifluoropropene further comprises HCl.

6. The process of claim 4, wherein the catalyst is activated by continuous or batch addition of anhydrous chlorine or chlorine.

7. The process of claim 1, wherein the hydrofluorination is vapor-phase fluorination or liquid-phase fluorination.

8. The process of claim 7, wherein the fluorination reaction is carried out at a temperature of between about 30° C to about 200° C, at a pressure of about 5 psia to about 200 psia.

9. The process of claim 4, wherein the mole ratio of hydrogen fluoride to 2-chloro-3,3,3-trifluoropropene is from about 1:1 to about 30:1.

10. The process of claim 9, wherein the mole ratio of hydrogen fluoride to 2-chloro-3,3,3-trifluoropropene is from about 2:1 to about 15:1.
